# EUROPEAN PATENT SPECIFICATION

(11) **EP 2 271 293 B1**
(45) Date of publication and mention of the grant of the patent: **28.10.2020**
(21) Application number: 09727947.5
(22) Date of filing: 01.04.2009
(51) Int. Cl.: A61F 6/14

(54) **AN INTRAUTERINE SYSTEM**
INTRAUTERINSYSTEM
SYSTÈME INTRA-UTÉRIN

(30) Priority: 02.04.2008 FI 20085277
(43) Date of publication of application: 12.01.2011
(73) Proprietor: Bayer Oy, 20210 Turku (FI)
(72) Inventor: KORTESUO, Pirjo, FI-21600 Parainen (FI); CALVO ALONSO, Ulla, FI-20760 Piispanristi (FI); INKI, Pirjo, FI-20780 Kaarina (FI); JUKARAINEN, Harri, FI-21620 Kuusisto (FI); JUTILA, Ilkka, FI-20660 Littoinen (FI); LEHTINEN, Juha, FI-20300 Turku (FI); LUKKARI-LAX, Eeva, FI-02180 Espoo (FI); LYYTIKÄINEN, Heikki, FI-21100 Naantali (FI); MOEDE, Joachim, FI-20700 Turku (FI); NIKANDER, Hannu, FI-21330 Paattinen (FI); SALLINEN, Pirjo, 10629 Berlin (DE); TJÄDER, Taina, FI-20660 Littoinen (FI); MacLEOD, Andrew, Cambridge Cambridgeshire CB22 7QT (GB); NOBLE, Michael, London Greater London E18 1PX (GB); WHITAKER, David, Harringay, Haringey London N8 0AN (GB)
(74) Representative: Laine IP Oy
(86) International application number: PCT/FI2009/050244
(87) International publication number: WO 2009/122016

(56) References cited:
- EP-A1- 0 036 805
- EP-A2- 0 873 751
- WO-A2-96/29026
- CN-A- 1 701 775
- CN-Y- 2 170 749
- NL-A- 8 601 570
- US-A- 3 467 087
- US-A- 3 834 378
- US-A- 3 938 515
- US-A1- 2006 089 658
- DATABASE WPI Week 198807, Derwent Publications Ltd., London, GB; AN 1988-047993, XP003025537 & NL 8 601 570 A (FUTURA NOVA BV) 18 January 1988

## Description

### Field of the invention

The present invention relates to intrauterine systems and to a method for manufacturing these systems.

### Background of the invention

The intrauterine systems, commonly known as IUS's, have long been known and they have been constructed in numerous shapes and sizes and of various materials. The conventional intrauterine systems consist normally of a plastic frame having the shape of the letter T or 7. The intrauterine systems containing drugs have been used to administer these drugs locally to the uterus at a controlled release rate over a prolonged period of time. Copper-releasing intrauterine devices as well as hormone releasing intrauterine systems have found considerable acceptance especially in contraception and hormonal treatment.

Intrauterine devices are described in several patents and patent applications.

US 3,952,734 by van Os et al. relates to an intrauterine device comprising an elongated stem having two resilient, cantilevered arms, extending sideways on either side of the stem. Due to the shape and flexibility the arms can easily be collapsed during the insertion step and relaxed again in the uterus to form part of an ellipse, the longer axis of which coincides with the stem. The stem may at least partially be covered with a layer preventing pregnancy, which layer enhances the effect of the device.

GB 1,282,618 and GB 1,405,763 by A H Robins CO relate to non-medicated intra-uterine contraceptive devices comprising a frame and a member, such as screening, grids, imperforate or perforated sheets, or one or more bars, attached to the frame along opposite edge parts of the internal periphery thereof, and cantilevered arms or spurs distributed around the ring and extending outwardly from the external periphery thereof for engaging the uterus wall and for impeding expulsion of the device.

EP 0873751 by Takeda Chemical Industries discloses a biodegradable IUD wherein an active agent is dispersed in a biodegradable polymer which is mould to a predetermined shape of a ring. Said IUD does not comprise separate frame and reservoir structures. As such systems are usually hard and inflexible, introduction of rings made of such material to the human body is very difficult. If the ring-like structure of the device is broken during the degradation process, it would be extremely difficult to remove the device because it would be deformed and its hard, broken parts would cause tissue damage.

WO 2003/017971 by Leiras Oy discloses intrauterine, intravaginal or intracervical drug delivery systems comprising a membrane and a core for the release of at least two active agents. Said systems are preferably T, 7, S, omega, ring or C shaped and do not comprise a closed continuous frame having a reservoir attached to it.

NL 8601570 by Futura Nova relates to an intrauterine device comprising an elongated stem which is combined to a ring of polymeric material. A contraceptive effect is achieved by covering the stem with a contraceptive material, preferably with metal and especially with copper in the form of a ring spiral on the stem. Said device does not comprise a separate reservoir consisting of a polymer matrix or polymer layer capable of controlling the release of the contraceptive material. Therefore the release rate of said contraceptive material could not be controlled but would depend on the solubility characteristics of the contraceptive. NL 8601570 disclose an intrauterine device according to the preamble of claim 1.

GB 1,318,554 by Michael Reese Hospital & Medical Center describes an intrauterine device comprising at least one capsule containing a progestin contained within a partially permeable wall but not dispersed in any polymer matrix. In one embodiment the device comprises three silicone elastomer tubes containing progestin and joined by polyethylene corner pieces to form a generally ring shaped or triangular device. The device is said to have sufficient rigidity to maintain its shape when not subjected to outside forces, but still be easily flexed as required for insertion. However, although the ends of the silicone tubes need not to be sharp, it is likely that they irritate uterine wall thus impairing wearing comfort.

GB 1,133,905 by Taylor relates to a mechanical intra-uterine contraceptive device comprising a closed loop of flexible material formed as two legs of substantially equal length intersecting at an apex end and a base joining the other ends of the legs, the intersection of the legs and the junctures of the legs and base being flexible and forming hinges whereby the contraceptive device can be collapsed for insertion into the uterus.

GB 1,116,916 by ORTHO PHARMA relates to an intra-uterine contraceptive device made of a resilient synthetic material and in the form of a torus or is of elongate shape, and comprises one or more elongate parts integral and intersecting with the elongate article, there being a web formed in the torus or at the junction of the intersecting parts to facilitate bending.

Many of the devices presented in the literature are bulky and/or rigid and may therefore cause side-effects and a high discontinuation rate. Undesirable complications that have been associated with the use of these intrauterine devices are pain and difficulties in insertion and/or in removal of the device, abdominal pain, infection, irregular bleeding, hormonal side effects, uterine perforation, cervical laceration, septic abortion, ectopic pregnancy, and expulsion of the IUS.

The insertion procedure can be uncomfortable or painful and sometimes causes cramps. With the devices that are drawn in the inserter tube prior to the insertion procedure, insertion pain is commonly related to the outer diameter of the insertion tube, which depends on the design and flexibility of said tube and the dimensions of the device to be inserted. With the devices where during the insertion procedure at least part of the device is outside the inserter tube, insertion pain is related to the outer diameter, design and flexibility of the insertion tube, but also to the size, design and flexibility of the device, especially of the part of the device laying outside the inserter tube. Inexperienced physician may also run into difficulties with insertion, but this can at least partly be overcome by training programs.

Pain soon after insertion usually occurs in the form of uterine cramps, and is probably related to uterine distention or irritation of the isthmic region caused by the device. The pain or discomfort is rarely present for more than the first weeks after the insertion.

Since most of the intrauterine systems are non-biodegradable, they will have to be removed after the treatment period, and depending on the device the removal may be difficult too and need quite some force. Abdominal pain and dysmenorrhoea are likely to be related to horizontal dimensions of the delivery system.

It is well known that the uterus contracts with a certain frequency continually and the contractions can push the device downward causing partial or complete expulsion. The contraction of the uterus will bring pressure on the inserted device. The transverse composition of forces will deform the device, and the longitudinal composition of forces will expel the device. The expulsion rate varies from less than one to more than 7 per 100 women in the first year of use and decreases with the parity and age. Expulsion is more common in younger women, who have never been pregnant or have never had children, or in women having an IUD inserted immediately after childbirth or abortion. Previous expulsion of an IUD, young age, hypermenorrhea, nulliparity and uterus sounding ≥9.0 cm have been associated with a higher rate of IUD dislocations. Correct insertion, with the IUD placed up to the fundus, is thought to reduce the chances of expulsion. Although the expulsion is not in itself a medical complication it is undesirable, because the IUS can then no longer provide protection against pregnancy.

Abnormal uterine bleeding after the insertion of a device occurs usually as intermenstrual bleeding or spotting. It results from the mechanical effects of the device on the uterine tissue, and may be increased with devices having pointed tips or sharp edges or excessively large size. A disparity between the size and shape of the uterine cavity and the device as well as inaccurate (non-fundal) placement of the device at the time of insertion have both been linked to increases in uterine bleeding. Abnormal bleeding, taking the form of menorrhagia, metrorrhagia, or both is perhaps the most common side effect of copper IUD's. The smaller sized IUDs usually cause less menstrual blood loss than the larger ones. After a certain period of time this side effect is not usually found with hormonal IUS's, which can be actually used for the treatment of menorrhagia, but particularly during the first six to seven cycles after insertion there are still undesired bleeding in about 15% of the women using the device. Bleeding is a medical indication for removal of the device only if it continues for more than 8 to 10 weeks or if it is severe enough to cause anaemia, but irregular bleeding is a common initial complaint among the users and often a reason for discontinuing the use of the system.

Perforation of the uterus is a serious condition that occurs in about 1 out of every 1000 women during the insertion and involves the uterine fundus or the cervix. Perforations may be partial, with only part of the IUD piercing the uterine wall or cervix, or complete the device passing through the uterus into the abdominal cavity. Bowel perforation and bowel obstruction as well as perforation of the urinary bladder and infertility due to adhesions have also been reported. Most perforations are thought to be associated with the insertion procedure, when the device itself or the sound or the inserter tube is accidentally pushed through the myometrium. Devices should be removed from the abdominal cavity because they can cause an inflammatory reaction and adhesions. There are several techniques for determining the presence and position of IUS's in the uterus and to exclude the possibility of perforation, for example by examining the strings of the device or by using ultrasound or fluoroscopic examination, hysteroscopy or abdominal x-ray. If the IUS has partially or fully perforated the uterus or cervix, the physician, by knowing the position of the IUS is better able to plan an appropriate strategy for removal of the IUS. It is important to keep in mind that, even when the strings are visible through the cervical os, perforation may have occurred.

A large number of different intrauterine devices have been proposed and applied in practice. The first IUDs that became generally used were large and extended the uterus and caused bleeding and pain, often accompanied by infections. There have been several attempts to overcome the disadvantages related to the intrauterine systems and devices have been designed with modifications aiming to decrease pain and bleeding, to make insertion and removal easier, to limit the risk of expulsion and especially to minimize the risk of perforation.

One basic approach has been to design and manufacture a large number of solid, intrauterine devices of varying sizes and assorted configurations for use in all types of uterine cavities. It is difficult to effectively design a shape of a device that would be satisfactory with a wide range of users. Also, varying the size of the device has been found to be inappropriate because of the lack of reliable techniques for determining the size of the uterine cavity. This would in many instances result in the wrong choice of device for insertion into the uterine cavity.

In an attempt to minimize the problem of expulsion, implantation technology in the form of frameless intrauterine devices has been developed. Frameless IUDs are said to be flexible and adaptable to the uterine cavities of every size and shape. However, since these devices are inserted and anchored into the myometrium of the uterine fundus, pain related to removal is hard to avoid.

Efforts to solve some of the issues related to the T-frames have been made by using more flexible material for the frame and especially for the horizontal arms of the frame, by modifying the angle between the arms and the vertical stem, by modifying the tips of the arms and by decreasing the size of the whole T-body, especially the diameter of the vertical arm to allow a thinner insertion tube.

The performance of an intrauterine system has been found to be determined largely by the interaction of the geometric parameters of the uterus and the device. The uterine cavity possesses a single axial and variable transverse and anteroposterior dimensions. Cyclic changes in uterine shape and size occur normally in women during different phases of the menstrual cycle. Larger size of an IUD has been stated to increase the risk of expulsion and side effects. Abnormalities in uterine geometry as a result of congenital or acquired space-occupying lesions reduce the uterine space available for IUDs and increase further the probability of IUD expulsion and other clinical complications.

Penetrating, anchoring mechanisms of the body of an IUD may cause more frequent and stronger contractions, bleeding, destroying of the mucosa, possible ascension of pathological germs and change of the local immune system. Devices can induce contractions, if a part of it irritates the tissue of the oviduct angles or the utero-tubal junctions (nervous complexes). The pointed tips of very thin or relatively rigid transverse or vertical arms of T-shaped devices have caused transverse uterine and retrograde cervical perforations (Hasson, BJOG, 89 (s4), 1-10, 1982).

Based on existing knowledge dimensions, design characteristics and material properties are important for an ideal intrauterine system, but the system should also be placed in a proper position in order to achieve the optimal contraceptive efficacy. In the case of T-shaped devices a small diameter of the vertical arm is essential to allow a thin insertion tube and the horizontal arms should be as smooth and flexible as possible.

Further, an ideal intrauterine system should be able to functionally adapt to the cyclic variations of the uterine cavity. The devices that are designed to fit to the size of the endometrial cavity are expected to have better performance records than those inserted at random, causing less irritation and less side effects (Kurz, Contraception. 1984 Jun;29(6):495-510) and producing less endometrial trauma and consequently less bleeding (Randic, Contracept Deliv Syst. 1980;1(2):87-94). The shape of the IUD should have blunt surfaces and gentle curves, and be devoid of sharp features which may cause uterine injury. Axial stiffness and transverse flexibility of the device appear to improve compliance properties (Hasson, BJOG, 89 (s4), 1-10, 1982).

Despite of the development work done, many intrauterine systems still have drawbacks. To overcome the issues related to various side effects described above and to improve patient compliance, new types of intrauterine systems have been introduced. The intrauterine systems according to present invention can be easily inserted in the stable optimal position in the uterus and are comfortable to use. They are flexible and have a smooth shape to minimize the risk of perforation, but still with low possibility for expulsions, and do not have any pain causing elements or structural features.

### Brief description of the figures

The invention is further illustrated by the following examples, describing various constructions of the intrauterine system according to the invention.

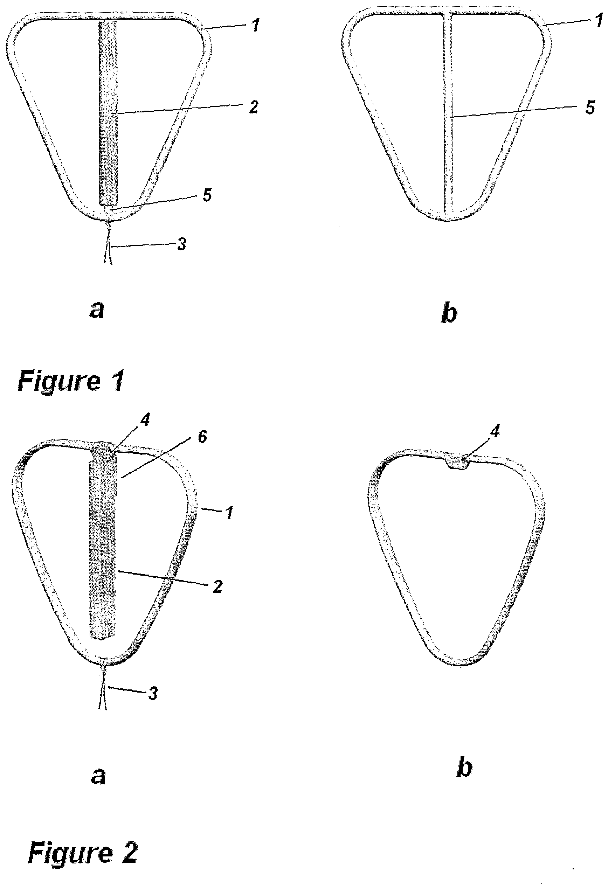

Figure 1 illustrates an intrauterine system (Figure 1a) and the corresponding frame (Figure 1b). The frame has a triangular shaped frame (1) with rounded corners. The reservoir (2) is assembled on the shaft (5) connected both to the lower and to the upper part of the frame.
Figure 2 illustrates an intrauterine system (Figure 2a) and the corresponding frame (Figure 2b). The frame (1) is a triangle with rounded corners and with flat cross section. The flat rectangular reservoir (2) is connected to the upper part of the frame by using a metal or polymer clip (6) and an extension (4) of the frame.

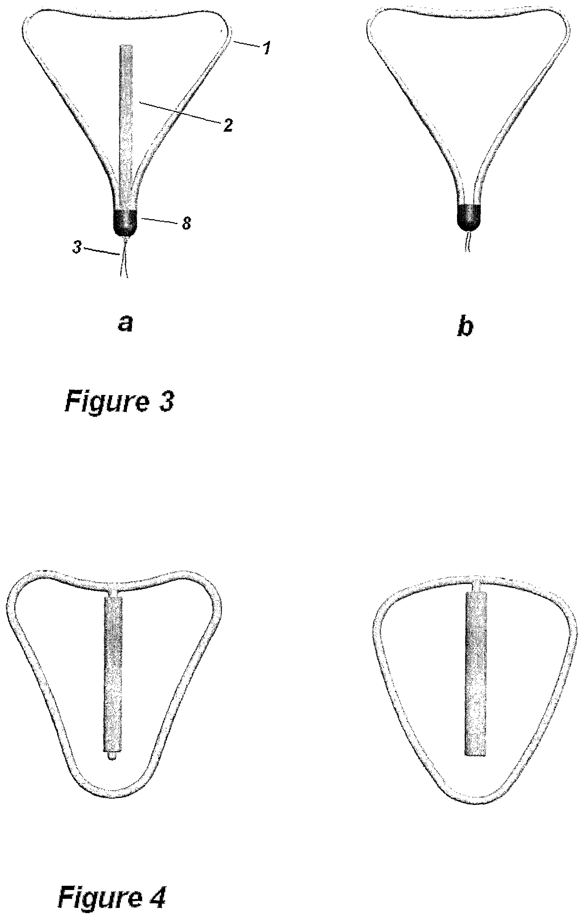

Figure 3 illustrates an intrauterine system having a frame (1) with a concave triangular shape and rounded corners. The reservoir (2) is placed inside the frame at the bottom apex and both are pushed into a polymer or metal cup (8). The threads (3) are passed through the hole in the bottom of the cup and knotted as close to the frame as possible. Figure 3b illustrates the frame.
Figure 4 illustrates further examples of different frames and reservoirs for the intrauterine systems according to the invention.

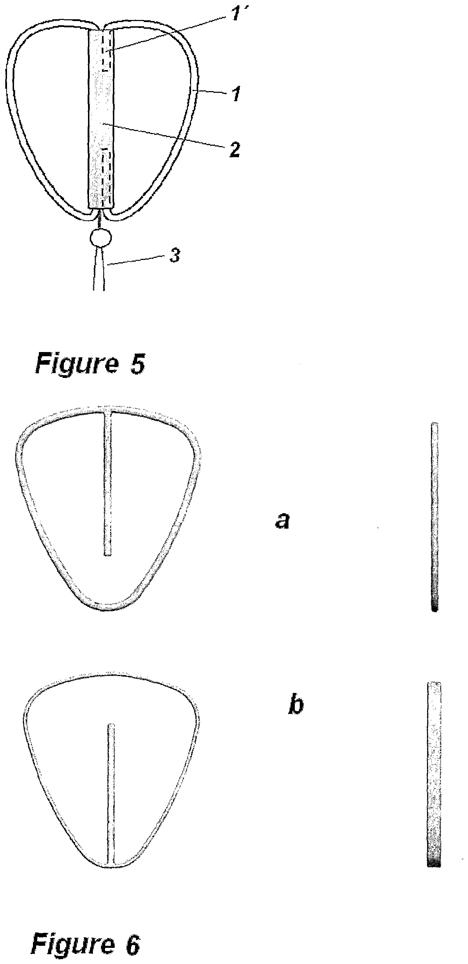

Figure 5 illustrates an intrauterine system wherein the ends of an open frame or frame halves (1') are used to attach the reservoir (2) to the frame. The threads (3) for the removal of the system are attached either at the lower end of the frame or inserted through the reservoir and attached at the upper end of the reservoir or of the frame.
Figure 6 illustrates a front and a side view of a triangular shaped intrauterine system having a frame with round cross section (Figure 6a) and flat cross section (Figure 6b).

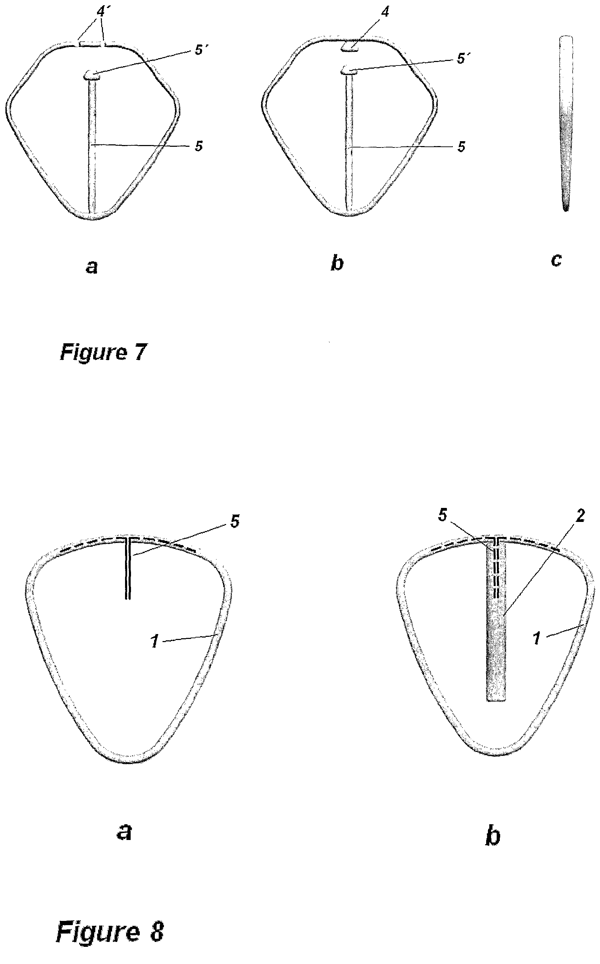

Figure 7 illustrates front view of pentagonal frames (Figure 7a and 7b) and a side view of the same frames (Figure 7c) showing local thinning at the lower part of the frame. Both frames have a shaft (5) connected to the bottom of the frame and a locking means (5') on the upper end of the shaft to retain the reservoir and prevent it from sliding off. The frame 7a has indentations (4') and the frame 7b an extension (4) on the upper part of the frame.
Figure 8 illustrates a triangular shaped frame (1, Figures 8a and 8b) comprising a metal or polymer supporting means inside the frame (5). The ends of the supporting means are bent to form a pair of rod like extensions or shafts on which the reservoir (2) is assembled.

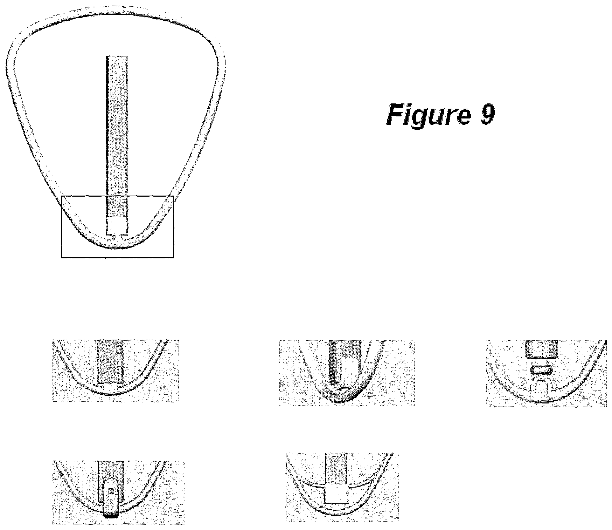

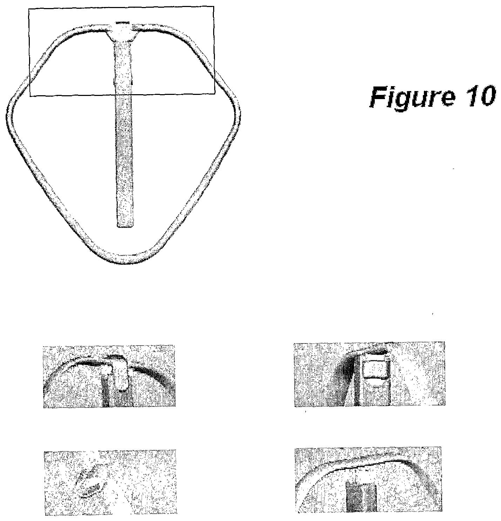

Figures 9 and 10 illustrate further examples of different methods to connect the reservoirs to the frame by using a metal or polymer insert, sleeve, supporting means, plug, staple, special clips, connectors, adapters, clothespin-type means or clamps or like.

### Object of the invention

The object of the present invention is an intra-uterine system (IUS) for a relatively long-term insertion into a uterine cavity, according to claim 1, and a method for manufacturing this type of intra-uterine system, according to claim 10 The reservoir connected to the frame gives the sufficient stiffness to the system, especially during the insertion step. Said frame and reservoir essentially comprise the same or different polymer composition,
Another object of the present invention is to provide an intrauterine system, which is easy to insert and remove without causing any pain, is easy and comfortable to use and has a shape and size fitting to the size of the endometrial cavity thus minimizing or eliminating the possibility of expulsion and avoiding side effects, for example such as caused by the irritation of the endometrium.

A further object of the invention is an intra-uterine system, which has a safe and optimized design to avoid the perforations or penetrations of the uterine wall.

Still another object of the invention is a convenient and reliable method according to claim 10, for delivering therapeutically active substances to a female mammal.

### Detailed description of the invention

The advantages of the invention are obtained by the intrauterine system as described above. The system comprises a frame and a reservoir connected to the frame, wherein the frame forms a continuous, closed and flexible system of polygonal shape and wherein at least one end of the reservoir is connected to the inner surface of the frame and the reservoir comprises at least one therapeutically active substance. The reservoir gives the sufficient stiffness to the intrauterine system during the insertion procedure and during the use. The frame is according to the invention, triangular or pentagonal. Said frame and reservoir essentially comprise the same or different polymer composition. The intrauterine system has an uncomplicated design and can be prepared by an economically attractive manufacturing process.

According to an embodiment, the invention provides an improved intrauterine system which is easy to insert and remove and is safe and comfortable to wear. The shape and size of the system are designed to fit to the size of the endometrial cavity and to avoid irritation of the endometrium, which usually would lead to various side effects and to discontinuation of the system.

According to another embodiment of the invention, the system has an optimized design and smooth shape to avoid the perforations or penetrations of the uterine wall.

The frame of the delivery system comprises a polymer composition and has a shape and size designed and adapted for placing in the endometrial cavity. The frame has a continuous, curved shape, which differs from a full circle by being pentagonal or triangular. The corners of polygonal frames are preferably slightly rounded. The frame may be coated by a polymer layer, a film or a membrane, said frame and polymer layer comprising the same or different polymer composition.

The frame is flexible and elastic. Flexible refers to the ability of the frame to bend easily and to withstand stress and strain without being damaged or broken. Stress is the force applied per unit area of a cross-section that causes deformation. Strain is the elongation or increase in the length relative to its original length. For example, the frame of the present invention can be deformed or flexed easily, such as by applying pressure from opposite external sides of the frame. Upon relieving of the pressure the frame will return to its original shape. Flexibility is particularly important and useful for enhancing user comfort while inserting, using or removing the intrauterine system.

The cross section of the frame can have almost any smooth shape, and can be for example circular, semi-circular, rectangular, oval, flat, elliptical, star-shaped, angular, polygonal and the like. The cross section may also vary along the length of the frame by having localised thinning, for example at the corners of polygonal, such as triangular or pentagonal, frames to adjust or further reduce the stiffness of the frame. The optimal shape and cross-section of the frame will render the system fundus seeking. The term fundus seeking means that instead of causing the expulsion of the system or changing the position of the system, the forces caused by the uterus or uterine contractions will at most only slightly push the system upwards, the main tension being balanced by the movement or vibration of the flexible frame.

The frame may comprise a supporting means, for example in a form of a core, fibre or wire, to reinforce the frame and/or to give additional flexibility to the frame. The supporting means can be made of any material which is inert and biologically compatible as long as it possesses sufficient strength and elasticity and remains unchanged for a sufficient period of time in the conditions prevailing in the uterus. Suitable stable biomedical materials for human use are well known in the art and include but are not limited to inert biocompatible metals, polymer composites, reinforced rubbers, flexible thermoplastic elastomers, such as ethyl vinyl acetate (EVA), thermoplastic polymers, such as styrene copolymers, for example styrene-isobutylene-styrene copolymer (SIBS) and styrene-butadiene-styrene copolymer (SBS), polyurethanes, thermoplastic urethane elastomers, thermoplastic polyurethane silicone elastomers, thermoplastic polyolefins, polyamides, polytetrafluoroethylene and polyethylenes. Biodegradable polymers can be used for contemporary supporting means.

The frame may also comprise means for attaching it into an inserter, for example a projection, a knob, a notch or an indentation.

The reservoir comprises at least one core, which is, according to the invention, encased by one or more polymer layers, either a membrane or a film. The length of the reservoir is preferably larger than the diameter or the width or height. The ends of the reservoir can be open or can be sealed by using for example an adhesive or the polymer composition of the membrane.

According to one embodiment of the invention the reservoir comprises one core encased by a polymer layer, either a membrane or a film, the core and the polymer layer essentially comprising the same or different polymer composition.

According to another embodiment of the invention the reservoir comprises two or more cores, each encased by a polymer layer, either a membrane or a film, said cores and polymer layers preferably comprising the same or different polymer composition.

According to still another embodiment of the invention, at least one of the cores of the reservoir comprises one or more therapeutically active agents to be delivered in the uterus.

The reservoir may have various sizes and shapes. Preferably the reservoir is a rod-like elongated element having for example circular, round, oval, flat, elliptical, rectangular, angular, polygonal or star-shaped cross section, and the like. The flat reservoir has a rectangular or essentially elliptical cross section. The corners or edges of the reservoir with rectangular, angular, polygonal or star-shaped cross section are preferably slightly rounded to avoid any sharp contact points which might irritate the uterus or reduce the wearing comfort. By choosing the flat shape the outer diameter of the reservoir and thus the dimensions of the inserter tube and/or the intrauterine system itself can be reduced. Reservoirs with unsymmetrical cross section, for example flat and rectangular reservoirs, can lie on the plane of the frame or perpendicular to that plane

According to the embodiment in which the reservoir comprises two or more cores, said cores may be positioned next to each other, side-by-side, one on the other or within each other. The length and the diameter of the cores may be the same or different. The cores can be separated from each other by a separation membrane or by an inert placebo core. One or more of the cores can also be a rod, a wire or a thread consisting of an inert biocompatible metal or of polymer, the purpose of which is to give additional rigidity and durability to the reservoir, and /or to serve to anchor or join the reservoir onto the frame. Any combination of structure is naturally possible and within the scope of the invention.

The polymer layer, a membrane or a film, may fully cover the frame, the supporting means or the core, or cover only a part of them, whereby the degree of extension can vary depending on a number of factors, for example such as the choice of materials. The thickness of the polymer layer depends for example on materials used as well as on the intended use of the intrauterine system. The membrane or film may consist of more than one layer in which case each layer has a certain thickness, and the thickness of the layers may be the same or different.

The intrauterine system may comprise a thread attachment, i.e. one or more threads or strings which can be used to remove or locate the system, or to detect the presence of the system if expulsion is to be suspected. Threads can be attached to the frame by several ways for example depending on whether the reservoir is connected to the top or to the bottom of the frame. When the reservoir is connected to the upper part of the frame, the threads are attached for example to the bottom of the frame, to the lower end of the reservoir or to both. Alternatively the threads can go through the reservoir to the upper part of the frame. When the reservoir is connected to the lower part of the frame, the threads are attached for example to the bottom of the frame or the threads can go through the reservoir to its upper end. In case the reservoir comprises one or more cores in the form of a thread, these threads can also be used as strings to detect or remove the intrauterine system after use or when necessary.

The intrauterine system according to the invention, either the frame or the reservoir, or both, may further comprise at least one image enhancing means to facilitate the detection of the device without a physical intrusion into the area of the body wherein the device has been inserted. The means can be for example X-ray contrast agent, a ferromagnetic agent or an agent for the ultrasound or fluoroscopic imaging of the system.

Said image enhancing means are preferably selected from the group consisting of
a) an inert metal coating on at least part of the body of the intrauterine system;
b) inert metal inserts, clips, rings or sleeves fixedly positioned on the body of the intrauterine system;
c) metal or ferromagnetic powder or particles or suitable metal or alkali metal salts mixed during the compounding step in the raw materials of the frame, core matrix or membrane of the intrauterine system, and
d) a metallic cup, connector, adapter, clamp, sleeve, shaft or holder fixed at a suitable position on the frame, which can also be used to anchor or join the reservoir onto the frame.

The metal is preferably selected from the group consisting of inert metals, such as silver, gold, titanium, tungsten, barium, bismuth, platinum, tantalum and palladium. Preferred metals are silver, gold, titanium and platinum, which are known to be compatible (i.e. physically inert) with the human body. However, copper may also be used.

Typically the thickness of the metal coating may vary from between about 0.1 nm and about 500 nm, preferably between about 1 nm and about 50 nm. However, even thicker coatings of about 0.1 mm are possible.

The metal clips, rings, sleeves or the like may be unembedded or at least partly embedded in the body of an IUS. Partial embedding of the metal parts smoothens the surface of the IUS while not yet impairing the sonographic visibility compared to unembedded counterparts. In case of rings it is advantageous to use double rings to enhance echogenicity. In case of clips and sleeves, the broader the clip or sleeve, the better is the visibility.

If metal powder, particles or salts are mixed with the raw materials of the body, core matrix or membrane of an IUS during the compounding step, the amount of metal powder is typically from about 0.1 to about 25% by weight, preferably from about 1 to about 10% by weight of the raw materials.

The intrauterine system according to the invention has been designed for a relatively long-term insertion into a uterine cavity. However, a long-term insertion may vary greatly, for example from a couple of weeks to several years, the time being typically from one to ten years, preferably from 1 to 5 years.

Polymer composition of the frame, the core, the membrane and the possible separation membrane or the inert placebo compartment, can be the same or different and may stand for one single polymer or a polymer composition, or may be made up of polymers that are blended with each other. In principle any polymer, either biodegradable or non-biodegradable, can be used as long as it is biocompatible. Further, the intrauterine system should retain structural integrity during the length of intended period of use.

Suitable materials are naturally occurring or synthetic materials, preferably materials that are biologically compatible with body fluids, and uterine tissues, and essentially insoluble in body fluids with which the device will come in contact. The use of rapidly dissolving materials or materials highly soluble in natural body fluids is to be avoided since the system is aimed to remain in place for prolonged periods of time.

The polymer material used must be flexible but have a relatively high degree of stiffness. The cross section thickness must be sufficiently high to provide wanted resilience in use, and this depends on the material used. However, the stiffness and the thickness must not be so high as to prevent the core or the frame from being bent through a substantial angle in use. Furthermore, it is important that the materials have a relatively high elasticity and characteristics which permit the device to be deformed and then again to return to its original configuration upon release of the deforming force.

Examples of suitable materials for the frame and the reservoir include, but are not limited to, polysiloxanes (typical examples thereof are available e.g. from Dow Corning and Nusil), poly (dimethyl siloxane) (PDMS), copolymers of dimethylsiloxanes, methylvinylsiloxanes, polyolefins such as polyethylene (available for example from Basell and Exxon), polypropylene, and polybutylenes; polyolefin copolymers, e.g., ethylenic copolymers such as ethylene vinyl acetate (EVA) copolymers, ethylene-methacrylic acid copolymers and ethylene-acrylic acid copolymers, ethylene/propylene copolymers, acrylic acid polymers, ethylene/ethyl acrylate copolymers, thermoplastic polyurethanes and thermoplastic polyurethane elastomers (available for example from Bayer Material Science and Lubrizol) including polyurethane copolymers, for example such as block and random copolymers that are polyether based, polyester based, polycarbonate based, aliphatic based, aromatic based and mixtures thereof, commercially available examples of which include Carbothane®, Tecoflex®, Tecothane®, Tecophilic®, Tecoplast®, Pellethane®, Chronothane® and Chronoflex®); thermoplastic polyurethane silicone elastomers (available for example from Aortech International), polycarbonates; polyurethanepolyureas, polyisocyanurates, polyurethane-polyisocyanurates, polyamide-polyurethanes, polybutadiene, polyisoprene, poly(methacrylate), polymethyl methacrylate, vinyl aromatic polymers such as polystyrene; vinyl aromatic copolymers such as copolymers of olefins and styrene or alpha-methyl styrene, for example, butadiene-styrene copolymers and copolymers of polyisobutylene with polystyrene or polymethylstyrene, for example styrene-isobutylene-styrene copolymer (SIBS) and styrene-butadiene-styrene copolymer (SBS) and polystyrene-polyisobutylene-polystyrene triblock copolymers, poly(hydroxyethylmethacrylate) (pHEMA), polyacetals; chloropolymers such as polyvinyl chloride (PVC); fluoropolymers such as polytetrafluoroethylene (PTFE); polyesters such as polyethyleneterephthalate (PET); polyester-ethers; polyamides such as nylon 6 and nylon 6,6; polyamide ethers such as polyether block amides (PEBA) comprising nylon blocks, polyvinyl acetate, polyacrylonitriles, polyethylene glycols, polymethylpentene, polyhydroxy alkanoates, for example such as poly(hydroxyvalerate), poly(hydroxybutyrate), poly(hydroxybutyrate-co-valerate), poly(lactic acids), poly(glycolic acids), poly(glycolide), poly(L-lactide), poly(lactide-co-glycolide), poly(glycolic acid-co-trimethylene carbonate), polyanhydrides, polyorthoesters, polyethers, polyether blocks, for example, poly(ethylene oxide), poly(trimethylene oxide), poly(propylene oxide) or poly(tetramethylene oxide) blocks, one specific example of which is a poly(tetramethylene oxide)- -polyamide-12 block copolymer (available from Elf Atochem as PEBAX), polyoctenamers such as Vestenamer®, a mixture of cyclic and linear polyoctenamers (available from Degussa Corp.), poly(caprolactone), poly(trimethylene carbonate), polyester amide, co-poly(ether-esters) (e.g. PEO/PLA), polyphosphazenes, biomolecules (such as fibrin, fibrinogen, cellulose, starch and collagen), hydrophilic polymers such as the hydrophilic hydrogels, cross-linked polyvinyl alcohol, neoprene rubber, butyl rubber, hydroxyl-terminated organopolysiloxanes of the room temperature vulcanizing type which harden to elastomers at room temperature following the addition of cross-linking agents in the presence of curing catalysts, one- or two-component dimethylpolysiloxane compositions cured by hydrosilylation at room temperature or under elevated temperatures, as well as mixtures thereof. According to the invention, the polymer composition comprises siloxane based elastomer, thermoplastic polyurethane, thermoplastic polyurethane elastomer, EVA, thermoplastic polyurethane silicone elastomer or a mixture of at least two of them.

Further exemplary naturally occurring or synthetic materials include such as poly(butylmethacrylate), plasticized nylon, plasticized soft nylon, plasticized poly(ethylene terephthalate), natural rubber, poly(isobutylene), poly(vinylidene chloride), cross-linked poly(vinylpyrrolidone), poly(trifluorochloroethylene), blends of poly(ethylene) and ethylene vinyl acetate copolymer, vinylidene chloride acrylonitrile, vinyl chloride diethyl fumarate, silicone rubbers, siliconecarbonate copolymers; poly(arylenes), poly(carbonates), ethylene-vinylalcohol copolymer, natural gum, polyalkylcyanoacrylate, carboxyvinyl polymer and collagen.

Preferred materials, especially for preparing the reservoir, the core and the membrane, are elastomer-forming silicone compositions which crosslink, for example upon heating, without production of volatile by-products. The absence of volatile by-products simplifies the manufacturing process by permitting a more accurate manufacture of the devices with respect to their shape and size. Due to the possibility of formulating compositions which crosslink at lower temperatures, the most preferred compositions are those silicone compositions which crosslink through reaction of unsaturated vinyl groups.

Especially advantageous are those compositions that comprise one or more organopolysiloxanes having per molecule at least two silicone-bonded groups having aliphatic unsaturation, an organosilicon cross-linking compound having at least two silicon-bonded hydrogen atoms and a catalyst e.g. a platinum compound or complex which promotes the reaction between unsaturated groups and silicon-bonded hydrogen groups. The platinum containing compound or complex is for example chloroplatinic acid, platinum acetylacetonate, a complex of platinum with unsaturated compounds such as ethylene, propylene, organovinylsiloxanes and styrene, me-thyldiplatinum and Pt(CN)3. The composition may include a catalyst inhibitor, for example an alkynyl compound, for example an acetylenically unsaturated secondary or tertiary alcohol such as ethynyl cyclohexanol. The aliphatically unsaturated groups are preferably terminally unsaturated.

The term "siloxane-based elastomer" shall be understood to cover elastomers made of poly (disubstituted siloxanes) where the substituents mainly are lower alkyl, preferably alkyl groups of 1 to 6 carbon atoms, or phenyl groups, wherein said alkyl or phenyl can be substituted or unsubstituted. A widely used and preferred polymer of this kind is poly(dimethylsiloxane) (PDMS).

The elastomer composition may also be selected from the group consisting of
- an elastomer composition comprising poly(dimethylsiloxane) (PDMS),
- an elastomer composition comprising a siloxane-based elastomer comprising 3,3,3-trifluoropropyl groups attached to the silicon atoms of the siloxane units,
- an elastomer composition comprising poly(alkylene oxide) groups, said poly(alkylene oxide) groups being present as alkoxy-terminated grafts or blocks linked to the polysiloxane units by silicon-carbon bonds or as a mixture of these forms, and
- a combination of at least two thereof.

According to a preferred embodiment of the invention, in the siloxane-based elastomer from 1 to approximately 50 % of the substituents attached to the silicon atoms of the siloxane units are 3,3,3-trifluoropropyl groups. The percentage of the substituents that are 3,3,3-trifluoropropyl groups can be for example 5-40 %, 10-35 %, 1-29 % or 15-49.5 %. One polymer of this kind, in which approximately 50 % of the methyl substituents at the silicon atoms are replaced by 3,3,3-trifluoropropyl groups, is commercially available. The term "approximately 50 %" means that the degree of 3,3,3-trifluoropropyl substitution is in fact somewhat below 50 %, because the polymer must contain a certain amount (about 0.15 % of the substituents) of cross-linkable groups such as vinyl or vinyl-terminated groups.

According to another preferred embodiment of the invention, the siloxane-based elastomer comprises poly(alkylene oxide) groups so that the poly(alkylene oxide) groups are present in the said elastomer either as alkoxy-terminated grafts of polysiloxane units or as blocks, the said grafts or blocks being linked to the polysiloxane units by silicon-carbon bonds. Preferably the poly(alkylene oxide) groups mentioned above are poly(ethylene oxide) (PEO) groups. In the core or membrane polymer composition the proportion of the polysiloxane comprising poly(alkylene oxide) groups, for example polydimethylsiloxane comprising poly(ethylene oxide) groups as alkoxy-terminated grafts or as blocks that are linked to the polysiloxane units by silicon-carbon bonds (PEO-b-PDMS copolymer) may vary from zero to 80 % of the total amount of polymers, but can naturally be higher.

The structural integrity of the material may be enhanced by the addition of a particulate material such as silica or diatomaceous earth. The elastomers can also be mixed with other additives to adjust elastomer's hydrophilic or hydrophobic properties while taking into account that all additives need to be biocompatible and harmless to the patient. The core or membrane may also comprise additional material to further adjust the release rate of one or several therapeutic substances, for example complex forming agents such as cyclodextrin derivatives to adjust the initial burst of the substance to the accepted or desired level or a fatty acid ester, preferably one containing from 2 to 20 carbon atoms. Auxiliary substances, for example such as tensides, anti-foaming agents, solubilisers or absorption retarders, or a mixture of any two or more of such substances, can also be added in order to impart the desired physical properties to the body of the delivery system. In addition, the polymer matrix may comprise other material, which can for example be used for identification or detection of the intrauterine system, such as metallic or magnetic particles or an X-ray contrast medium like barium sulphate.

Any suitable design of the delivery system or any combination of structure is naturally possible and within the scope of the invention.

Insertion forces of intrauterine devices (IUDs) and systems (IUSs) have been found to depend on the material and dimensions of the device, design characteristics such as the contour of the leading edge, and on the inserter design, dimensions and material properties. The forces caused by the removal process of the device have been observed to depend on the dimensions, flexibility and design of the IUS.

The above mentioned forces can be translated into pain during insertion and removal as well as into wearing comfort during the use of the system. Furthermore, the dimensions and material of the IUS are also believed to affect the wearing comfort of the IUS when it is placed in the uterus. In addition, a too large size of an IUD relative to the uterine cavity is associated with increased risk of complications, such as expulsion of the IUD or increased bleeding.

Preliminary tests done with the intrauterine systems of the present invention confirmed that existing relatively simple training models for pelvic anatomy provided insufficient realism and that suitable phantoms for female reproductive anatomy did not exist. Therefore, to test the properties of the intrauterine systems and to provide scientific basis for evaluating and developing optimal construction and design of these systems in order to achieve maximum wearing comfort and appropriate positioning of the system in the uterus, computer assisted virtual modelling was used and relevant functional laboratory test models were developed.

The model with typical female pelvic anatomy, including material features which give the tactile feed-back similar to that from *in vivo* situation, was designed and manufactured by moulding the inner parts by using appropriate polymers to give as realistic sensation as possible. Interchangeable cervix and uterus elements having different sizes and shapes, as well as a variety of positions (anteversion, retroversion) were used to adjust and exchange the anatomy and to allow simulation of the full range of female pelvic anatomy. Also the flexion, *i.e.* the hinge region between the uterine cervix and the uterine body, could be adjusted to allow comparison of insertion and removal forces representing the pain during these procedures, respectively.

With the test models typical anatomical features that impact the device's critical features in terms of insertion and removal forces as well as forces exerted from being placed *in situ* in the uterus could be simulated. Test models also made possible to allow modifications to simulate extreme anatomical situations, and to compare the properties and behaviour of these systems to the existing intrauterine devices and intrauterine systems. The models also enabled *in-vitro* set up for repeatable relative attribute testing with the possibility to include animal tissue for absolute testing.

The pressure caused by the intrauterine system on the uterus walls and the cervix was evaluated by using laboratory test model and computer assisted virtual modelling. Correct positioning and the tendency to expulsion can be deduced based on the relative forces the system exerts on the fundus, uterine walls and cervix.

Experiments done with laboratory test models confirmed that insertion forces did primarily depend on the dimensions, design characteristics and on material properties of the intrauterine system. The forces needed for the removal of the intrauterine system, representing the propensity of the system to expulsion, depend on the dimensions, flexibility and design of the IUS.

The intrauterine systems according to present invention have a body with blunt surfaces and gentle curves without any sharp features which would cause uterine injury. Therefore they especially fulfil the requirements for an ideal intrauterine system.

According to the invention, the intrauterine systems having a continuous, closed frame were found to exert relatively low pressures, suggesting that these are more comfortable than most existing intrauterine devices and systems. Intrauterine systems having for example more natural uterus shaped frame appear in the simulation tests to be fundus seeking as opposed to the systems having essentially round shaped frames. Especially polygonal such as triangular and pentagonal, as well as shield shaped and almond shaped frames, i.e. those frames which taper towards the cervix generally exert a greater proportion of overall force on the fundus, thus having a very low or no tendency to expulsion. In addition, these frames have reduced projection into utero-tubal junctions and therefore do not irritate uterine walls at all.

Rounder shapes which have a tendency to extend or elongate downwards or in both directions and exert pressure on the cervix, have higher tendency to expulsion. The size of the intrauterine system is naturally an important factor. Polygonal frames having rounded corners, for example almond and shield, which were intentionally modelled too large for the uterus, have a propensity to elongate and apply pressure to both the fundus and the cervix. Some fundus seeking frames, although they do not exert pressure on the cervix, may apply a high force on the upper uterus walls, especially if the upper part of the frame is very rigid or relatively large. This problem impairing compliance properties can be overcome by optimizing the size of the frame and selecting suitable material for the frame. A flatter cross section of the polygonal frame, as opposed to substantially round cross section, tend to increase the device memory and the opening force and give also rise to lower pressures on the uterus suggesting that a design based on this shape could exhibit both fundus seeking and high compliance properties. Further, variable cross section of the frame, for example with localised thinning at the corners of a polygonal frame can be used to reduce stiffness.

### Manufacturing methods

The intrauterine systems in accordance with the invention can be prepared by methods well known in the art. A variety of thermoplastic processing techniques may be used including for example extrusion techniques, such as extrusion, co-extrusion, multi-layer extrusion, multi-lumen extrusion, and so on, and molding techniques, such as rotational molding and injection molding including co-injection or sequential injection molding technology, laminar injection molding, where multilayer structures are desired, compression or any other appropriate methods known in the art. The desired geometry of the intrauterine system can be achieved by using appropriately sized and shaped moulds or extrusion dies. The frame and the reservoir may be manufactured separately followed by their assembly, simultaneously or sequentially.

Injection molding of one or more polymeric materials, and thermoset materials in particular, can be used to efficiently produce a frame, a reservoir comprising a membrane and a core, optionally containing an active agent, or a complete intrauterine system comprising a frame and a reservoir. The polymer composition may be injected into a mold cavity of desired shape sequentially with one or more injection nozzles or syringes, or simultaneously by using a co-injection nozzle having two axially symmetric openings. The mold may be capable of producing more than one article in a given injection cycle by the use of multiple mold cavities. The molds and mold designs are well known in the art, and may be selected or adapted to produce the desired physical shape of the product.

Another preferred method of manufacture comprises extrusion. Selected polymer composition is extruded through a suitable die to form a rod-like or tube-like extrudate having desired diameter and shape of the cross section. The fibre is cut into pieces having an appropriate length required to form the frame, the reservoir or the supporting means for the frame, each having a desired size and shape. The pieces may then be assembled in any manner by using different methods suitable for this purpose, for example by placing the piece or pieces in a mould which has a desired form to produce a rod-like reservoir having one or more cores, or a continuous closed frame described above. The ends of the extruded pieces can be appropriately joined together by using a coupling means.

The coupling means can be any method, mechanism, device or material known in the art for bonding materials or structures together. Exemplary coupling means include for example injection molding, welding techniques, such as the hot-gas welding technique well known in the art, solvent bonding, adhesive joining, use of a layer of uncured, cross linkable elastomer forming composition, heat fusing, heat bonding, pressure, and the like. When manufacturing the frame, the polymeric substance must be sufficiently pliable when dry to allow the rods to be bent and formed into the final shape of the frame.

Tubular frame elements can also be joined into a closed system by using a plug or a stopper made of an inert, biocompatible material. Examples of suitable material are metals, such as gold, silver or silver alloys, tantalum, platinum, glass or ceramic material or any suitable polymers. If desired, a biocompatible adhesive can be used for better sealing or better adhesion of the plug or stopper to the frame element.

The polymer layer, a membrane or a film, can be applied onto the frame, core or the set of cores according to known methods such as by using extrusion or injection moulding methods, coating, spraying or dipping. Discontinuous coating can be used to produce reservoirs with sealed ends. As an alternative, the prefabricated membrane tube can be used. The tube is first expanded mechanically for example with a suitable device or by using for example pressurized gas, such as air, or by swelling the tube in a suitable solvent, such as cyclohexane, diglyme, isopropanol, or in a mixture of solvents, where after the swollen membrane tube is mounted onto the core. When the solvent evaporates, the membrane tightens on the core.

The reservoirs comprising several cores or the frame element consisting of more than one segment, can also be prepared for example by using a coextrusion method described in the Finnish patent FI 97947. Polymer or polymer composition is processed to the desired shape and size by using known extrusion methods. The membrane layer may then be applied onto the prefabricated suitably sized cores by feeding each of the cores to the extruder followed either by another core or by an empty space filled with air, which during the extrusion process will be filled with the membrane material.

The support means can be of solid material or hollow and can be prepared in a similar way.

The reservoir can practically be at any point inside the frame, at least one end of the reservoir being connected to any point on the inner surface of the frame by using several alternative methods. To achieve a simple insertion, the reservoir is preferably attached to the upper or lower part of the frame, or to both parts. The frame or the reservoir comprises retention or locking means to fix and retain the reservoir and to prevent it from sliding off.

The reservoir can be fixed on the frame by using different methods. The frame may for example comprise an elongated extension in the form of a metal or polymer shaft, core, rod or pin or the like at a suitable point on which the hollow tube-like reservoir is assembled, preferably by first enlarging the diameter of the reservoir tube to some degree, for example by using pressure or solvent swelling, and thereafter by simply sliding the reservoir onto the extension or inserting the extension into the hollow reservoir. The extension is preferably flexible in order to facilitate the assembly of the reservoir on it. After the reservoir has been assembled, the free end of the elongated extension may be for example heat formed to create a physical retention feature to mechanically retain the reservoir and prevent it from sliding off. To keep the reservoir in place the extension may also comprise a suitably shaped locking means or a stopper, over which the swollen reservoir is inserted.

The frame may also comprise a metal or polymer supporting means which is bent at the ends to form rod-like extensions on which the reservoir is assembled or molded. The ends of an open frame or frame halves can be inserted into the reservoir to join the reservoir and the frame together thus simultaneously forming the intrauterine system having a continuous closed frame. The ends of an open frame can also be bent to form extensions, on which the reservoir is assembled or molded. Further, the reservoir can be manufactured by coating the extension with a polymer layer, containing a therapeutically active substance, by using injection molding, dipping, spraying and like.

Other methods to attach the reservoir to the frame include for example known techniques of welding, use of an adhesive, or use of special metal or polymer inserts, clips, connectors, adapters, clothespin-type means or clamps or like. The intrauterine system can also be manufactured by using a metal or polymer cup, plug or sleeve which mechanically retains the reservoir, the threads and the frame or the ends of an open frame element. The cup, plug or sleeve could be rifled on the inner surface to reduce 'stiction' and to allow easier detachment. In this case threads preferably protrude through the base of the cup. These methods are especially suitable to be used with solid reservoirs, i.e. the reservoirs not having a hollow, tube-like structure. A complete intrauterine system can further be manufactured by using for example injection molding techniques.

The frame according to the invention can principally be manufactured in any size as required. For optimal performance and wearing comfort the exact size depends on the mammal and particular application, and the size should be such that the system would not have a tendency to move or rotate inside the average sized uterine cavity. For human female an outer diameter of the frame is typically from 18 to 42 mm, preferably from 20 to 38 mm or from 22 to 36 mm. The cross sectional diameter is typically from 0.5 to 10 mm, preferably from 1 to 6 mm and more preferably from about 1.5 to 4 mm.

The dimensions of the reservoir depend on the application in which the intrauterine system is to be used. The dimensions of a drug containing delivery system depend on the expected release rate of the therapeutically active substance and the expected life-time of the intrauterine system. Typically the outer diameter of the reservoir, or the height and the width in case of a flat or a rectangular reservoir, may vary from 0.5 to 5 mm, preferably from 1 to 3.5 mm. If the reservoir is manufactured by coating methods, the wall thickness can be from 0.01 to about 5 mm, preferably from 0.2 to 3.5 mm. The length of the reservoir may vary from 0.5 mm up to the internal diameter of the frame, preferably from 15 to 36 mm.

The thickness of the polymer layer, the membrane or the film, encasing the core is such that it can be manufactured within acceptable tolerances by methods known in the art and conveniently lies within the range of from 0.01 to 1.0 mm, preferably from 0.1 to 0.6 mm. The thickness of a polymer layer separating the cores can be about from 0.01 to 5 mm.

The intrauterine delivery systems in accordance with the invention can be manufactured aseptically or can be sterilized by using known methods, for example by using physical, chemical or technical sterilization.

The frame is preferably manufactured by injection molding using known methods and tools having suitable shape and size. The reservoir according to the present invention can be easily fabricated in accordance with standard techniques. Once the polymer composition of core or cores has been selected, the desired shape of the reservoir is achieved for example by molding, casting extrusion, or by other appropriate processes. When the core material comprises polymers such as silicone elastomers, an additional curing step may be necessary. The membrane or film layer is then applied to the thus shaped core by using an appropriate method discussed above, e.g., by swelling a prefabricated polymer tube in a suitable solvent or by using mechanical stretching,, placing it over the core and allowing the polymer to dry in place, or by dipping, wrapping, spraying, laminating or according to other known techniques.

A therapeutically active substance in a finely ground or even micronized form will be mixed in the polymer material of the core prior to processing to achieve a substantially uniform dispersion. A person skilled in the art is readily able to choose the geometry of the device and the polymer composition so that the desired daily release of the at least one pharmacologically active agent is achieved, and to determine the amount of the therapeutically active agent needed for each specific application and for the desired time of duration. Here the term "geometry" of the device primarily and specifically encompasses the overall dimensions and shape of the reservoir, i.e. the cross-sectional diameter, or the height and the width, as well as the length.

A variety of different therapeutically active or prophylactic substances can be used in conjunction with the invention. By "therapeutically active substance" is meant any substance or a salt, an ester or a prodrug thereof which by administration in the uterus is capable of defending against, or treating, a disease state in the human or animal body. By "prophylactic substance" is meant any substance (or its salt or prodrug) effective in defending against a disease state in the human or animal body, preferably the human body. The active substance(s) may be hydrophilic or lipophilic material(s).

Suitable therapeutically active or prophylactic substances for use in the present invention include, but are not limited to, the following: hormones, steroids, contraceptive drugs, drugs for hormone replacement therapy, selective androgen receptor modulators (SARM), drugs for the treatment of premenstrual syndrome, drugs for the treatment of endometriosis, drugs for the treatment of uterine fibroids (uterine leiomyomata and leiomyosarcoma), drugs for cervical ripening / induction of labour, selective estrogen receptor modulators (SERMs), selective progestin receptor modulators (SPRM), antimalarial substances, osteoporosis drugs, antiprogestins, aromatase inhibitors, bone active substances, anti-urinary incontinence substances, serotonin reuptake inhibitors (SSRIs), drugs for genito-urinary disorders, antiemetic drugs, 5HT3 antagonists, anti-angiogenesis factors, growth factors, enzymes, anesthetics, analgesics, anticoagulants and thrombolytic substances, antiinflammatory substances, antimicrobials, anti-protozoal substances, antiviral substances, neuroleptic and antipsychotic drugs, opiate antagonists and agonists, anti-fibroid substances, antihypertensives, angiotensin inhibitors, anti-protozoal substances, anti-addiction drugs, anti-angiogenesis factors, anti-bacterial substances, anticancer chemotherapeutic substances, antifungals, antioxidants, diuretics, drugs for the central nervous system, fibrinolytic substances, free radical scavengers, gene therapy substances, growth factors, neurotrophic factors, peptides, photodynamic therapy substances, proteins, symphatomimetic substances, thrombin inhibitors, thrombolytic substances, and a combination of at least two thereof.

Therapeutically active substances especially suitable for use in the present invention include gestagenes selected from the group of levonorgestrel, norgestimat, norethisteron, dydrogesteron, drospirenon, 3-beta-hydroxydesogestrel, 3-ketodesogestrel (= etonogestrel), 17-deacetylnorgestimat, 19-norprogesteron, acetoxypregnenolon, allylestrenol, amgeston, chlormadinon, cyproteron, demegeston, desogestrel, dieno-gest, dihydrogesteron, dimethisteron, ethisteron, ethynodioldiacetat, flurogestonacetat, gastrinon, gestoden, gestrinon, hydroxymethylprogesteron, hydroxyprogesteron, lynestrenol (= lynoestrenol), mecirogeston, medroxyprogesteron, megestrol, melengestrol, nomegestrol, norethindron (= norethisteron), norethynodrel, norgestrel (including d-norgestrel und dl-norgestrel), norgestrienon, normethisteron, progesteron, quingestanol, (17alpha)-17-hydroxy-ll-methylen-19-norpregna-4,15-dien-20-yn-3-on, tibolon, trimegeston, algeston acetophenid, nestoron, promegeston, 17-hydroxyprogesteronester, 19-nor-17hydroxyprogesteron, 17alpha-ethinyl-testosteron, 17alpha-ethinyl-19-nor-testosteron, d-17beta-acetoxy-13beta-ethyl-17alpha-ethinyl-gon-4-en-3-onoxim, tanaproget, or estrogenes selected from the group ethinylestradiol, mestranol, quinestranol, estradiol, estron, estran, estriol, estetrol, conjugated equine estrogenes.

The amount of the therapeutically active substance incorporated in the reservoir of the delivery system varies depending on the particular therapeutically active substance, the desired therapeutic effect and the time for which the system is expected to provide therapy. Reservoirs with varying sizes and shapes can be formulated for administering dosages for different therapeutical areas. The upper limit on the amount of therapeutically active substance depends on the size of the reservoir. The lower limit depends on the activity of the therapeutically active substance and on the expected release time. A person skilled in the art is readily able to determine the amount of the therapeutically active substance needed for each specific application of the delivery system. Preferably, the amount of therapeutically active substance varies between almost zero to 70 wt-%, when it is mixed into the polymer composition, the preferred amount being between 20 - 60 wt-%. Other possible ranges of the amount of the therapeutically active substance are 0.5-70 wt-%, 5-65 wt-%, 10-50 wt-%, 25-70 wt-%, 50-60 wt-% and 40-50 wt-%.

Based on the above, a further object of the invention is a method for manufacturing an intrauterine system having a closed continuous frame and a reservoir connected to the frame, said method comprising of injection molding, extruding or compressing the frame and the reservoir simultaneously, or by using a sequential process comprising the steps of preparing the frame, preparing the first composition comprising a therapeutically active agent and a polymer composition to provide a core, preparing the second composition comprising a polymer composition to provide a membrane encasing the core, combining the core and the membrane to produce a reservoir, and connecting together the reservoir and the frame.

### Mechanical testing of frames

The mechanical properties of the intrauterine systems, and especially of the frame, must ensure optimal uterine compatibility and user acceptability. If the mechanical strength is too low, the system could either be expulsed from the uterus or be prone to rupture. If the mechanical strength is too high, the inflexibility of the device could cause irritation or ulceration of the uterine tissue. Therefore the mechanical characteristics, flexibility and memory of the frames were assessed by using standard methods of compressing described in the literature. Flexibility is tested for characterising the property of a frame to resist low and moderate short term deformation. Memory is measured for characterising the ability of a frame to recover its shape after acute compaction.

The invention is further illustrated by the following examples.

### Example 1

### Core preparation

98.8 parts by weight of poly(dimethylsiloxane-co-vinylmethylsiloxane) and 1.2 parts by weight of dichlorobenzoylperoxide-polydimethylsiloxane paste (50 % of dichlorobenzoylperoxide) are mixed with a 2-roll mill. The mixture is extruded to form a rod with an outer diameter of 1,8 mm and cured by heat at + 150 °C for 15 minutes, during which crosslinking takes place. The crosslinked core is cut into 23 mm length.

### Membrane preparation

100 parts by weight of silica-filled poly(trifluoropropylmethylsiloxane-co-vinylmethylsiloxane), in which the content of trifluoropropyl-methylsiloxane units was 99 mol-%; i.e. degree of trifluoropropyl substitution is 49.5 %, and 1.2 parts by weight of dichlorobenzoylperoxide-polydimethylsiloxane paste (50 % of dichlorobenzoylperoxide) are mixed with a 2-roll mill. The mixture is extruded into a tube-like form with a wall thickness of 0.22 mm and cured by heat.

### Preparation of the reservoir

The membrane tube of 25 mm is swelled with cyclohexane and pulled over the core. Cyclohexane is allowed to evaporate. The ends of the reservoir are closed with a silicone adhesive.

### Example 2

### Core preparation

The core having the length of 18 mm is prepared according to Example 1.

### Membrane preparation

99 parts of silica-filled poly(dimethylsiloxane-co-vinylmethylsiloxane), 10 ppm Pt-catalyst (of the reactant) and 0.03 parts of inhibitor (ethynyl cyclohexanol) and approximately 0.6 parts of poly(hydrogenmethylsiloxane-co-dimethylsiloxane) crosslinker are mixed in a 2-roll mill. The membrane material is extruded to a tube-like form with a wall thickness of 0.3 mm and cured by heat.

### Example 3

### Core preparation

99.6 parts of commercial poly(dimethylsiloxane-co-vinylmethylsiloxane), 0.4 parts of poly(hydrogenmethylsiloxane-co-dimethylsiloxane) crosslinker, 0.02 parts of ethynyl cyclohexanol inhibitor and 10 ppm of Pt-catalyst (of the reactant) in vinyl-methyl-siloxane are mixed in a kneating mill. The mixture is extruded to a tube-like form with a wall thickness of 0.7 mm and an outer diameter of 2.6 mm. The extrudate is cured by heat at +115 °C for 30 minutes, cooled and cut to the length of 30 mm.

### Membrane preparation

9 parts of α,ω-divinylether terminated poly(ethylene oxide)-b-poly(dimethylsiloxane) multiblock copolymer (PEO-b-PDMS), 89 parts of silica-filled poly(dimethylsiloxane-co-vinylmethylsiloxane), 10 ppm Pt-catalyst (of the reactant), 0.03 parts inhibitor (ethynyl cyclohexanol), and approximately 2 parts of poly(hydrogenmethylsiloxane-co-dimethylsiloxane) crosslinker are mixed in a two-roll mill. The mixture is extruded to a tube-like form with a wall thickness of 0.15 mm and cured by heat.

### Preparation of the intrauterine system

The membrane tube having the length of 3.1 mm is swollen in isopropanol and pulled over the core. Cyclohexane is allowed to evaporate. Thereafter the reservoir is swollen in isopropanol and pulled over the elongated extension of the frame comprising thermoplastic polyurethane composition.. Cyclohexane is again allowed to evaporate.

### Example 4

### Core preparation

48.5 parts of PEO-b-PDMS, 49 parts of poly(dimethylsiloxane-covinylmethylsiloxane), 10 ppm Pt-catalyst (of the reactant), 0.02 parts inhibitor (ethynyl cyclohexanol), and approximately 2.4 parts of poly(hydrogenmethylsiloxane-co-dimethylsiloxane) crosslinker are mixed in a two-roll mill. The mixture is extruded to form a rod with an outer diameter of 2.1 mm and cured by heat at + 150 °C for 15 minutes, during which crosslinking takes place. The crosslinked core is cut into the length of 15 mm.

The second core is prepared according to Example 3. The crosslinked core having an outer diameter of 2.1 mm is cut into the length of 10 mm.

### Preparation of the membrane and the reservoir

9 parts of PEO-b-PDMS, 89 parts of silica-filled poly(dimethylsiloxane-co-vinylmethyl-siloxane), 10 ppm Pt-catalyst (of the reactant), 0.03 parts inhibitor (ethynyl cyclohexanol), and approximately 2 parts of poly(hydrogenmethylsiloxane-co-dimethyl-siloxane) crosslinker are mixed in a two-roll mill. The membrane material is coating extruded on the above prepared two cores by successively inserting them through the inner nozzle. The formed wall thickness of the membrane is 0.2 mm.

### Preparation of the intrauterine system

The thread is first looped around the triangular frame and the ends of the thread are then passed through the hole in the bottom of a silver cup. Next the reservoir is placed inside the frame at the bottom apex. The bottom apex of the frame with the reservoir is pushed into the silver cup and the threads pulled tight to ensure that three parts are located and a knot tied to secure the assembly. The threads are then trimmed to the appropriate length.

### Example 5

### Core preparation

48.5 parts of PEO-b-PDMS, 49 parts of poly(dimethylsiloxane-covinylmethylsiloxane), 10 ppm Pt-catalyst (of the reactant), 0.02 parts inhibitor (ethynyl cyclohexanol), and approximately 2.4 parts of poly(hydrogenmethylsiloxane-co-dimethylsiloxane) crosslinker are mixed in a two-roll mill. The mixture is extruded to form a flat, rectangular rod with slightly rounded corners and cured by heat at + 150 °C for 15 minutes, during which crosslinking takes place. The outer diameters of the core are 0.9 mm (height) and 2.1 mm (width). The crosslinked core is cut into the length of 22 mm.

### Preparation of the intrauterine system

To prepare the reservoir, the core is dip-coated by a PDMS membrane having a wall thickness of 0.22 mm. A pentagonal frame with rounded corners is prepared of thermoplastic polyurethane by injection molding. The reservoir is connected to the upper part of the frame by using a modified electrical connector. The top of the clip or connector loops over the frame and is tightened around the frame. The other end, the tab, is trapped behind the reservoir which is held in place by the jaws of the connector wound around the reservoir.

### Example 6

### Core preparation

54 parts of commercial poly(dimethylsiloxane-co-vinylmethylsiloxane), 45.5 parts by weight of levonorgestrel, 0.4 parts of poly(hydrogenmethylsiloxane-co-dimethylsiloxane) crosslinker, 0.02 parts of ethynyl cyclohexanol inhibitor and 10 ppm of Pt-catalyst (of the reaction species) in vinyl-methyl-siloxane were mixed in a kneating mill. The mixture was extruded and cured by heat at + 115 °C for 30 minutes and cooled. The crosslinked core having an outer diameter of 2.2 mm was cut into 20 mm length.

### Membrane preparation

27 parts of α,ω-divinylether terminated poly(ethylene oxide)-b-poly(dimethylsiloxane) multiblock copolymer (PEO-b-PDMS), 71 parts of silica-filled poly(dimethylsiloxane-co-vinylmethylsiloxane), 10 ppm Pt-catalyst (of the reaction species), 0.03 parts inhibitor (ethynyl cyclohexanol), and approximately 2 parts of poly(hydrogenmethylsiloxane-co-dimethylsiloxane) crosslinker were mixed in a two-roll mill. The mixture was extruded to a tube-like form with a wall thickness of 0.22 mm and cured by heat.

### Preparation of the delivery system

The membrane was swollen in isopropanol and pulled over the core. The reservoir so formed was attached into a metal clip fixed tightly at the lower part of the pentagonal frame comprising thermoplastic polyurethane elastomer.

### Example 7

### Preparation of the intrauterine system comprising the frame, the reservoir, a silver ring and the removal thread

The frame is injection moulded. Molten thermoplastic is injected at high pressure into a mould, which is the inverse of the frame shape. The moulded frame is ejected from the tool and when it is cooled down the gate and spigot are removed and any flash is trimmed off. Next the silver ring and the prefabricated tube-like reservoir are assembled onto the central shaft of the frame. The free end of the central shaft is then heat formed to create a physical retention feature to mechanically retain the reservoir and prevent it from sliding off. Next the thread is looped through the frame and secured with a knot. The threads are then trimmed to the appropriate length.

## Claims

1. An intra-uterine system for a long-term insertion into a uterine cavity, **characterized in that** said intra-uterine system comprises a reservoir (2) and a continuous, closed and flexible frame (1), wherein
(a) at least one end of the reservoir (2) is connected to the inner surface of the frame (1) and
(b) the reservoir (2) comprises at least one core comprising at least one therapeutically active substance and
(c) the frame (1) and the reservoir (2) comprise a flexible polymer composition comprising siloxane based elastomer, thermoplastic polyurethane, thermoplastic polyurethane elastomer, EVA, polyethylene, thermoplastic polyurethane silicone elastomer or a mixture of at least two of them, **characterised in that** the flexible frame (1) is of triangular or pentagonal shape and a polymer layer encases said at least one core.

2. An intra-uterine system according to claim 1, wherein the polymer composition of the frame (1) and the reservoir (2) is the same or different.

3. An intra-uterine system according to any one of claims 1-2, wherein the cross section of the frame (1) is circular, semi-circular, oval, flat, elliptical, rectangular, angular, polygonal or star-shaped.

4. An intra-uterine system according to any of claims 1-3, wherein the cross section of the reservoir (2) is circular, oval, flat, elliptical, rectangular, angular, polygonal or star-shaped.

5. An intra-uterine system according to any of claims 1-4, wherein the frame (1) comprises a supporting means consisting of a polymer composition or a biocompatible metal.

6. An intrauterine system according to any of claims 1-5, wherein it comprises threads (3) for removal, location or detection of the system.

7. An intrauterine system according to any of claims 1-6, wherein said system comprises at least one image enhancing means for improving the detection and/or location of the of the system.

8. An intrauterine system according to claim 7, wherein the image enhancing means are selected from the group consisting of
a) an inert metal coating on at least part of the body of the intrauterine system;
b) inert metal inserts, clips, rings or sleeves fixedly positioned on the body of the intrauterine system;
c) metal or ferromagnetic powder or particles or suitable metal or alkali metal salts mixed during the compounding step in the raw materials of the frame (1), core or membrane of the intrauterine system, and
d) a metallic cup, connector, adapter, clamp, sleeve or holder fixed at a suitable position on the frame (1), which can also be used to anchor or join the reservoir (2) onto the frame (1).

9. An intrauterine system according to any of claims 1-8, wherein the frame (1) or the reservoir (2) comprises retention or locking means (5') to retain the reservoir and to prevent it from sliding off.

10. A method for manufacturing an intrauterine system, according to any of claims 1-9, having a closed continuous and flexible frame (1) of triangular or pentagonal shape and a reservoir (2) comprising at least one core, which comprises at least one therapeutically active substance and a membrane encasing the core, which reservoir (2) is connected to the inner surface of the frame (1), said method comprising injection molding, extruding or compressing the frame (1) and the reservoir (2) simultaneously, or by using a sequential process comprising the steps of preparing the frame (1), preparing the first composition comprising a therapeutically active substance and a polymer composition to provide the core, preparing the second composition comprising a polymer composition to provide the membrane encasing the core, combining the core and the membrane to produce a reservoir (2), and connecting together the reservoir (2) and the frame (1).

## Patentansprüche

1. Intrauterinsystem für eine langfristige Einführung in eine Gebärmutterhöhle, **dadurch gekennzeichnet, dass** das Intrauterinsystem ein Reservoir (2) und einen kontinuierlichen, geschlossenen und flexiblen Rahmen (1) umfasst, wobei
(a) mindestens ein Ende des Reservoirs (2) mit der Innenfläche des Rahmens (1) verbunden ist und
(b) das Reservoir (2) mindestens einen Kern umfasst, der mindestens eine therapeutisch wirksame Substanz umfasst, und
(c) der Rahmen (1) und das Reservoir (2) eine flexible Polymerzusammensetzung umfassen, die ein Elastomer auf Siloxanbasis, thermoplastisches Polyurethan, thermoplastisches Polyurethanelastomer, EVA, Polyethylen, thermoplastisches Polyurethan-Silikonelastomer oder ein Gemisch von mindestens zwei von ihnen umfasst, **dadurch gekennzeichnet, dass** der flexible Rahmen (1) eine dreieckige oder fünfeckige Form aufweist und eine Polymerschicht den mindestens einen Kern umhüllt.

2. Intrauterinsystem nach Anspruch 1, wobei die Polymerzusammensetzung des Rahmens (1) und des Reservoirs (2) gleich oder unterschiedlich ist.

3. Intrauterinsystem nach einem der Ansprüche 1-2, wobei der Querschnitt des Rahmens (1) kreisförmig, halbkreisförmig, oval, flach, elliptisch, rechteckig, winklig, polygonal oder sternförmig ist.

4. Intrauterinsystem nach einem der Ansprüche 1-3, wobei der Querschnitt des Reservoirs (2) kreisförmig, oval, flach, elliptisch, rechteckig, winklig, polygonal oder sternförmig ist.

5. Intrauterinsystem nach einem der Ansprüche 1-4, wobei der Rahmen (1) ein Stützmittel umfasst, das aus einer Polymerzusammensetzung oder einem biokompatiblen Metall besteht.

6. Intrauterinsystem nach einem der Ansprüche 1-5, wobei es Fäden (3) zur Entfernung, Lokalisierung oder zum Nachweis des Systems umfasst.

7. Intrauterinsystem nach einem der Ansprüche 1-6, wobei das System mindestens ein bildverbesserndes Mittel zur Verbesserung des Nachweises und/oder der Lokalisierung des Systems umfasst.

8. Intrauterinsystem nach Anspruch 7, wobei die bildverbessernden Mittel ausgewählt sind aus der Gruppe, bestehend aus
a) einer inerten Metallbeschichtung auf mindestens einem Teil des Körpers des Intrauterinsystems;
b) inerten Metalleinsätzen, Clips, Ringen oder Hülsen, die fest auf dem Körper des Intrauterinsystems positioniert sind;
c) Metall- oder ferromagnetischem Pulver oder Partikeln oder geeigneten Metall- oder Alkalimetallsalzen, die während des Compoundierungsschritts in die Rohmaterialien des Rahmens (1), Kerns oder der Membran des Intrauterinsystems gemischt werden, und
d) einem metallischen Becher, Verbinder, Adapter, Klemme, Hülse oder Halter, der an einer geeigneten Position am Rahmen (1) befestigt ist, der auch zur Verankerung oder Verbindung des Reservoirs (2) mit dem Rahmen (1) verwendet werden kann.

9. Intrauterinsystem nach einem der Ansprüche 1-8, wobei der Rahmen (1) oder das Reservoir (2) Rückhalte- oder Verriegelungsmittel (5') umfasst, um das Reservoir zurückzuhalten und zu verhindern, dass es abrutscht.

10. Verfahren zur Herstellung eines Intrauterinsystems nach einem der Ansprüche 1-9 mit einem geschlossenen, kontinuierlichen und flexiblen Rahmen (1) von dreieckiger oder fünfeckiger Form und einem Reservoir (2), das mindestens einen Kern, der mindestens eine therapeutisch wirksame Substanz umfasst, und eine den Kern umschließende Membran umfasst, wobei das Reservoir (2) mit der Innenfläche des Rahmens (1) verbunden ist, wobei das Verfahren das Spritzgießen, Extrudieren oder Komprimieren des Rahmens (1) und des Reservoirs (2) gleichzeitig umfasst, oder durch Verwendung eines sequentiellen Verfahrens, umfassend die Schritte des Herstellens des Rahmens (1), des Herstellens der ersten Zusammensetzung, umfassend eine therapeutisch wirksame Substanz und eine Polymerzusammensetzung, um den Kern bereitzustellen, des Herstellens der zweiten Zusammensetzung, umfassend eine Polymerzusammensetzung, um die Membran bereitzustellen, die den Kern umhüllt, des Kombinierens des Kerns und der Membran, um ein Reservoir (2) zu erzeugen, und des Verbindens des Reservoirs (2) und des Rahmens (1) miteinander.

## Revendications

1. Système intra-utérin pour une insertion à long terme dans une cavité utérine, **caractérisé en ce que** ledit système intra-utérin comprend un réservoir (2) et un cadre continu, fermé et souple (1), dans lequel
(a) au moins une extrémité du réservoir (2) est reliée à la surface intérieure du cadre (1) et
(b) le réservoir (2) comprend au moins un cœur comprenant au moins une substance thérapeutiquement active, et
(c) le cadre (1) et le réservoir (2) comprennent une composition polymère souple comprenant un élastomère à base de siloxane, un polyuréthane thermoplastique, un élastomère de polyuréthane thermoplastique, un EVA, un polyéthylène, un élastomère de silicone polyuréthane thermoplastique ou un mélange d'au moins deux d'entre eux, **caractérisé en ce que** le cadre (1) souple est de forme triangulaire ou pentagonale et une couche de polymère recouvre ledit au moins un cœur.

2. Système intra-utérin selon la revendication 1, dans lequel la composition polymère du cadre (1) et du réservoir (2) est identique ou différente.

3. Système intra-utérin selon l'une quelconque des revendications 1-2, dans lequel la section transversale du cadre (1) est circulaire, semi-circulaire, ovale, plate, elliptique, rectangulaire, angulaire, polygonale ou en forme d'étoile.

4. Système intra-utérin selon l'une quelconque des revendications 1-3, dans lequel la section transversale du réservoir (2) est circulaire, ovale, plate, elliptique, rectangulaire, angulaire, polygonale ou en forme d'étoile.

5. Système intra-utérin selon l'une quelconque des revendications 1-4, dans lequel le cadre (1) comprend un moyen de support constituant en une composition polymère ou un métal biocompatible.

6. Système intra-utérin selon l'une quelconque des revendications 1-5, dans lequel il comprend des fils (3) pour le retrait, la localisation ou la détection du système.

7. Système intra-utérin selon l'une quelconque des revendications 1-6, dans lequel ledit système comprend au moins un moyen d'amélioration d'image pour améliorer la détection et/ou la localisation du système.

8. Système intra-utérin selon la revendication 7, dans lequel les moyens d'amélioration d'image sont choisis dans le groupe consistant en
a) un revêtement en métal inerte sur au moins une partie du corps du système intra-utérin ;
b) des inserts, agrafes, anneaux ou manchons en métal inerte positionnés de manière fixe sur le corps du système intra-utérin ;
c) une poudre ou des particules métalliques ou ferromagnétiques ou des sels métalliques ou alcalino-métalliques appropriés mélangés pendant l'étape de mélange dans les matières premières du cadre (1), du cœur ou de la membrane du système intra-utérin, et
d) une coupelle, un connecteur, un adaptateur, une pince, un manchon ou un support métallique fixé au niveau d'une position appropriée sur le cadre (1), qui peut également être utilisé(e) pour ancrer ou joindre le réservoir (2) sur le cadre (1).

9. Système intra-utérin selon l'une quelconque des revendications 1-8, dans lequel le cadre (1) ou le réservoir (2) comprend un moyen de retenue ou de verrouillage (5') pour retenir le réservoir et l'empêcher de glisser.

10. Procédé de fabrication d'un système intra-utérin selon l'une quelconque des revendications 1-9, ayant un cadre (1) fermé, continu et souple de forme triangulaire ou polygonale et un réservoir (2) comprenant au moins un cœur, qui comprend au moins une substance thérapeutiquement active et une membrane recouvrant le cœur, lequel réservoir (2) est relié à la surface intérieure du cadre (1), ledit procédé comprenant le moulage par injection, l'extrusion ou la compression du cadre (1) et du réservoir (2) simultanément, ou en utilisant un procédé séquentiel comprenant les étapes de préparation du cadre (1), préparation de la première composition comprenant une substance thérapeutiquement active et une composition polymère pour fournir le cœur, préparation de la seconde composition comprenant une composition polymère pour fournir une membrane recouvrant le cœur, combinaison du cœur et de la membrane pour produire un réservoir (2), et le fait de relier ensemble le réservoir (2) et le cadre (1).
